# EUROPÄISCHE PATENTSCHRIFT

(11) **EP 1 103 226 B1**
(45) Veröffentlichungstag und Bekanntmachung des Hinweises auf die Patenterteilung: **17.01.2007**
(21) Anmeldenummer: 00122129.0
(22) Anmeldetag: 12.10.2000
(51) Int. Cl.: A61B 17/70

(54) **Querverbinder**
Transverse connector
Connecteur transversal

(30) Priorität: 29.11.1999 DE 19957332
(43) Veröffentlichungstag der Anmeldung: 30.05.2001
(73) Patentinhaber: DePuy Spine Sàrl, 2400 Le Locle (CH)
(72) Erfinder: Schäfer, Bernd, 6315 Oberägeri (CH)
(74) Vertreter: Dreiss, Fuhlendorf, Steimle & Becker

(56) Entgegenhaltungen:
- EP-A- 0 737 448
- EP-A- 0 784 963
- EP-A- 0 956 829
- FR-A- 2 697 743
- US-A- 5 562 663

## Beschreibung

Die Erfindung betrifft einen Querverbinder für die Osteosynthese mit den Merkmalen des Oberbegriffs des Anspruchs 1.

Es ist bekannt, dass Wirbelsäulenverkrümmungen dadurch behandelt werden, dass mehrere Wirbel über einen Fixierstab miteinander verbunden und dadurch lagekorrigiert werden. Hierfür werden in die Wirbel Pedikelschrauben eingedreht bzw. auf die Wirbel Knochenplatten aufgesetzt, in welche der Fixierstab oder ggf. auch mehrere Fixierstäbe eingesetzt werden. Bei extremen Wirbelsäulenverkrümmungen kommt es mitunter jedoch vor, dass die Krümmung des Fixierstabes nicht ausreicht, um die Wirbel bzw. die in den Wirbel eingeschraubten Knochenschrauben bzw. aufgesetzten Wirbelplatten an den Fixierstab anzubinden. Für derartige Wirbelsäulenverkrümmungen müssen in der Regel mehrere Fixierstäbe verwendet werden, die aneinandergesetzt werden. Dies führt jedoch dazu, dass in die einzelnen Wirbel mehrere Knochenschrauben eingedreht werden müssen, was mitunter zu Problemen führt, da die Festigkeit der Wirbel darunter leidet.

Aus der EP-A-0 737 448, die den nächstkommenden Stand der Technik darstellt, ist ein Querverbinder bekannt, bei dem der Klemmabschnitt mit einem Klemmdeckel verschlossen wird, der Klemmdeckel ein loses Bauteil darstellt und deshalb die Gefahr besteht, dass er bei der Operation verloren geht. Die US-A-5,562,663 zeigt einen ähnlich gestalteten Klemmabschnitt mit Klemmdeckel. Die FR-A-2,697,743 offenbart einen Querverbinder mit einem Aufnahmeauge für einen Querstab, der mittels radial einzudrehender Schrauben fixiert wird. Diese Art der Fixierung lässt keine große Kraftübertragung zu.

Der Erfindung liegt daher die Aufgabe zugrunde, einen Querverbinder bereitzustellen, mit dem auch extrem gekrümmte Wirbelsäulen korrigiert werden können und der einfach zu handhaben ist.

Diese Aufgabe wird erfindungsgemäß mit einem Querverbinder gelöst, der die Merkmale des Anspruchs 1 aufweist.

Mit dem erfindungsgemäßen Querverbinder besteht nun die Möglichkeit, auch bei extrem gekrümmten Wirbelsäulen Fixierstäbe zu verwenden, die weniger gebogen sind, wobei die Fixierstäbe über den erfindungsgemäßen Querverbinder mit den Knochenschrauben bzw. Knochenplatten und somit mit den Wirbeln verbunden werden, wenn eine direkte Anbindung des Fixierstabes an den Knochenschrauben bzw. Knochenplatten nicht möglich ist. Durch den erfindungsgemäßen Querverbinder können also die Korrekturkräfte sicher und spielfrei vom Fixierstab auf die entsprechenden Wirbel übertragen werden. Über den stabförmigen Abschnitt wird der Querverbinder entweder an der Knochenschraube oder an der Knochenplatte befestigt, wobei mittels des Klemmabschnitts der Fixierstab am Querverbinder befestigt wird. Der Klemmdeckel besitzt einen den stabförmigen Abschnitt des Querverbinders umgreifenden Bügel. Auf diese Weise wird der Klemmdeckel verliersicher gehalten. Außerdem werden über den Bügel die Klemm- und Haltekräfte des Klemmdeckels auf den stabförmigen Abschnitt übertragen.

Bei einer Weiterbildung ist vorgesehen, dass der Klemmabschnitt eine den Fixierstab aufnehmende Öffnung aufweist und diese Öffnung mit einem randoffenen Schlitz versehen ist. Durch die Öffnung wird der Fixierstab hindurchgeführt und dadurch gehalten, dass der Querschnitt der Öffnung nach dem Einsetzen des Fixierstabes verkleinert wird. Dies erfolgt üblicherweise dadurch, dass eine Schraube eingedreht wird, die den Schlitz verkleinert und dadurch den Fixierstab in der Aufnahmeöffnung verklemmt. Es besteht jedoch auch die Möglichkeit, dass eine Fixierschraube in den Klemmabschnitt eingeschraubt wird, die direkt am Fixierstab angreift und diesen hält. Derartige Klemmschrauben sind in der Regel als Madenschrauben ausgebildet.

Dabei weist der Klemmdeckel einen Teil der Aufnahmeöffnung für den Fixierstab auf. Eine derartige Ausgestaltung des Klemmabschnitts besitzt den wesentlichen Vorteil, dass der Fixierstab relativ einfach in die Aufnahmeöffnung eingesetzt werden kann und erst nach dem Einsetzen bzw. Einlegen des Fixierstabes der Klemmdeckel aufgesetzt und dadurch die Aufnahmeöffnung verschlossen wird. Dabei ist der Klemmdeckel bevorzugt verschwenkbar am stabförmigen Abschnitt gelagert. Durch Aufklappen kann der Fixierstab relativ einfach entnommen und durch das Zuklappen des Klemmdeckels wird die Aufnahmeöffnung, wie bereits erwähnt, nach dem Einsetzen des Fixierstabes geschlossen.

Eine Ausführungsform sieht vor, dass der Bügel aus der Ebene des Klemmdeckels im Wesentlichen um 90° abgewinkelt ist und den stabförmigen Abschnitt orthogonal zu dessen Längsachse umgreift. Diese Ausgestaltung des Bügels erlaubt zum einen das Aufschwenken bzw. Aufklappen des Klemmdeckels, zum anderen eine optimale Krafteinleitung und Halterung des Klemmdeckels am stabförmigen Abschnitt.

Um eine definierte Lage des Klemmdeckels zu schaffen, weist der Bügel eine ein Lager bildende Schulter auf, die sich an einem Gegenlager des anderen Teils des Klemmabschnitts abstützt. Über diese beiden Lagerteile werden auch die beiden Teile der Aufnahmeöffnung, in welche der Fixierstab eingelegt wird, zueinander ausgerichtet.

Mit Vorzug sind einerseits das Lager mit Gegenlager und andererseits eine Klemmschraube auf gegenüberliegenden Seiten der Aufnahmeöffnung für den Fixierstab angeordnet. Auf der Seite des Lagers mit Gegenlager befindet sich außerdem der Bügel, der den stabförmigen Abschnitt umgreift. Diese Ausgestaltung gewährleistet, dass auch hohe Kräfte vom Fixierstab in den Querverbinder problemlos eingeleitet werden können, ohne dass dadurch eine Positionsänderung des Fixierstabes im Klemmabschnitt erfolgt.

Eine weitere Sicherung gegen Verrutschen und/oder Verdrehen des Querverbinders in der Knochenschraube bzw. der Knochenplatte wird dadurch erzielt, dass der stabförmige Abschnitt eine Oberflächenstruktur aufweist, die insbesondere mit in Längsrichtung verlaufenden Nuten versehen ist. Außerdem kann die Aufnahmeöffnung für den Fixierstab an ihrer Innenoberfläche zumindest abschnittsweise mit einer Oberflächenstruktur, insbesondere mit in Längsrichtung zur Öffnung verlaufenden Nuten versehen sein. Diese Oberflächenstruktur verhindert eine Positionsänderung des Bauteils im zugehörigen Klemmelement. Andere Oberflächenstrukturen, wie eine Riffelung, eine Gewindestruktur usw., sind ebenfalls denkbar.

Um problemlos an den gleichen Knochenschrauben und/oder Knochenplatten sowohl Fixierstäbe als auch den erfindungsgemäßen Querverbinder verwenden zu können, entspricht der Durchmesser des stabförmigen Abschnitts dem Durchmesser der Aufnahmeöffnung für den Fixierstab. Somit bedarf es keiner speziellen Knochenschrauben und/oder Knochenplatten für den Querverbinder.

Eine Vereinfachung des chirurgischen Eingriffs wird dadurch erzielt, dass der Klemmabschnitt mit einer verliersicher befestigten Schraube versehen ist. Es besteht nicht mehr die Gefahr, dass nach dem Einsetzen des Querverbinders, jedoch vor dem endgültigen Verklemmen des Fixierstabes Teile des Querverbinders abhanden kommen. Die Schraube, mit welcher der Klemmdeckel befestigt wird, ist stets verliersicher am Klemmdeckel gehalten.

Weitere Vorteile, Merkmale und Einzelheiten der Erfindung ergeben sich aus den Unteransprüchen sowie der nachfolgenden Beschreibung, in der unter Bezugnahme auf die Zeichnung ein besonders bevorzugtes Ausführungsbeispiel im Einzelnen beschrieben ist. Dabei können die in der Zeichnung dargestellten als auch in den Ansprüchen und in der Beschreibung erwähnten Merkmale jeweils einzeln für sich oder in beliebiger Kombination erfindungswesentlich sein. In der Zeichnung zeigen:
- Figur 1: eine Seitenansicht des erfindungsgemäßen Querverbinders;
- Figur 2: eine Draufsicht auf den Querverbinder in Richtung des Pfeils II gemäß Figur 1; und
- Figur 3: eine perspektivische Ansicht des Querverbinders.

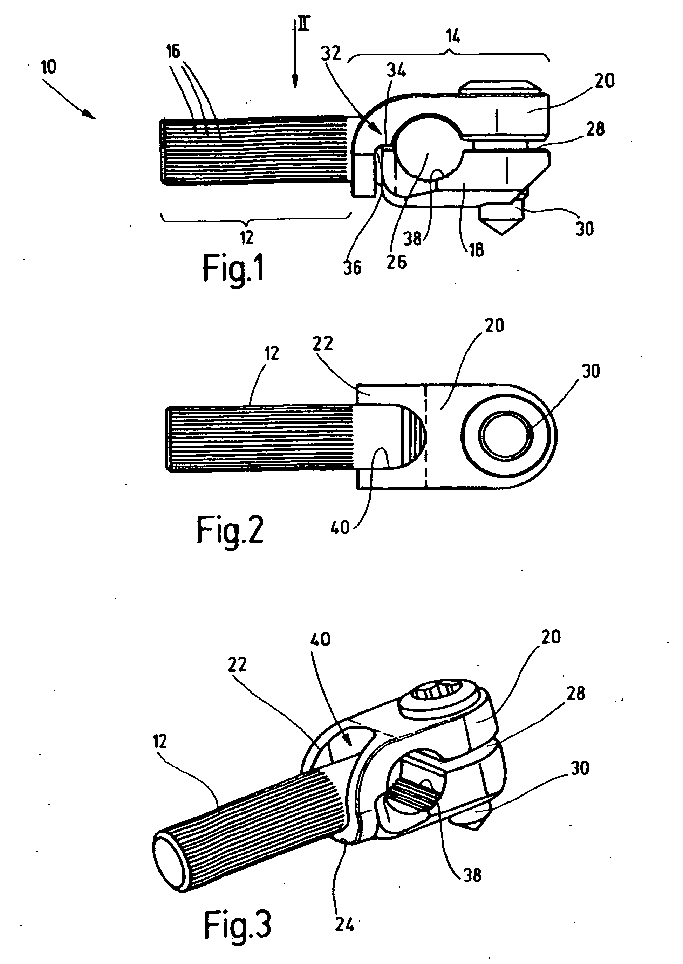

Die Figur 1 zeigt ein bevorzugtes Ausführungsbeispiel eines Querverbinders 10 gemäß der Erfindung. Dieser Querverbinder 10 wird von einem stabförmigen Abschnitt 12 und einem Klemmabschnitt 14 gebildet. Am stabförmigen Abschnitt 12 wird der Querverbinder 10 z.B. an einer (nicht dargestellten) Knochenschraube oder an einer (nicht dargestellten) Knochenplatte befestigt. Dabei entspricht der Querschnitt des stabförmigen Abschnitts 12 im Wesentlichen dem Querschnitt eines (nicht dargestellten) Fixierstabes, mit dem Wirbelsäulenverkrümmungen korrigiert werden. Dieser stabförmige Abschnitt 12 besitzt einen runden Querschnitt mit an der Außenseite in Längsrichtung verlaufende Nuten 16, die eine Verdrehsicherung darstellen.

Der Klemmabschnitt 14 ist zweiteilig aufgebaut und besitzt einen ersten Teil 18, der einstückig mit dem stabförmigen Abschnitt 12 verbunden ist. Der zweite Teil ist als Klemmdeckel 20 ausgebildet und verschwenkbar am stabförmigen Abschnitt 12 gelagert. Hierfür besitzt der Klemmdeckel 20, was insbesondere deutlich aus Fig. 3 ersichtlich ist, einen den stabförmigen Abschnitt 12 umgreifenden Bügel 22, der aus der Ebene des Klemmdeckels 20 bogenförmig um 90° abgewinkelt ist und mit seinem dem Klemmdeckel 20 entfernten Ende 24 im Wesentlichen orthogonal zur Längsachse des stabförmigen Abschnitts 12 steht.

Der Klemmdeckel 20 und der erste Teil 18 des Klemmabschnitts 14 bilden eine Aufnahmeöffnung 26, die mit einem randoffenen Schlitz 28 versehen ist. In die Aufnahmeöffnung 26 wird der (nicht dargestellt) Fixierstab eingelegt und durch Verklemmen befestigt. Das Verklemmen erfolgt dadurch, dass eine Klemmschraube 30, die verliersicher im Klemmdeckel 20 gehalten wird, in eine entsprechende Gewindeöffnung im ersten Teil 18 eingeschraubt wird. Mit der Klemmschraube 30 wird der Klemmdeckel 20 auf den Fixierstab aufgeklemmt. Dabei verschwenkt der Klemmdeckel 12 um ein Schwenklager 32, welches von einer Schulter 34 und einem Gegenlager 36 gebildet wird.

Um den Fixierstab stabil im Klemmabschnitt 14 fixieren zu können, weist die Aufnahmeöffnung 26 in deren Achsrichtung verlaufende Längsnuten 38 auf, die an einem Teil des Innenumfangs der Aufnahmeöffnung 26 vorgesehen sind.

Ein problemloses Einlegen des Fixierstabes in den Klemmabschnitt 14, selbst bei an einer Knochenschraube befestigtem Querverbinder 10, erfolgt dadurch, dass nach dem Lösen der Klemmschraube 30 der Schwenkdeckel um das Schwenklager 32 aufgeschwenkt wird. Dabei verschwenkt der stabförmige Abschnitt 12 in einer vom Bügel 22 umgebenen, nierenförmigen Öffnung 40. Der Fixierstab kann nun problemlos in die offene Aufnahmeöffnung 26 eingelegt werden. Die Fixierung erfolgt durch Zuklappen des Klemmdeckels 20 und Befestigen der Klemmschraube 30 am ersten Teil 18.

Bei einer in der Zeichnung nicht dargestellten Ausführungsform ist der Klemmabschnitt 14 einteilig ausgestaltet, d.h. der Klemmdeckel 20 am ersten Teil 18 einstückig angeformt. Die Aufnahmeöffnung 26 wird dadurch verjüngt und ein darin eingelegter Klemmstab gehalten, indem der Klemmabschnitt 14 durch Einschrauben der Klemmschraube 30 elastisch verformt wird. Bei diesem Ausführungsbeispiel muss der Querverbinder 10 auf den Fixierstab aufgeschoben werden, bevor dieser an den Knochenschrauben oder Knochenplatten fixiert wird.

## Patentansprüche

1. Querverbinder für die Osteosynthese zur Anbindung eines mehrere Wirbel miteinander verbindenden Fixierstabes, wobei der Querverbinder (10) einen stabförmigen Abschnitt (12) zu dessen Befestigung und einen Klemmabschnitt (14) zur Anbindung des Fixierstabes aufweist und der Klemmabschnitt (14) zweiteilig mit einem Klemmdeckel (20) ausgebildet ist, **dadurch gekennzeichnet, dass** der Klemmdeckel (20) mit einem den stabförmigen Abschnitt (12) umgreifenden Bügel (22) versehen ist.

2. Querverbinder nach Anspruch 1, **dadurch gekennzeichnet, dass** der Klemmabschnitt (14) eine den Fixierstab aufnehmende Öffnung (26) aufweist und diese Öffnung (26) mit einem randoffenen Schlitz (28) versehen ist.

3. Querverbinder nach Anspruch 1 oder 2, **dadurch gekennzeichnet, dass** der Klemmdeckel (20) mit einem Teil der Aufnahmeöffnung (26) für den Fixierstab ausgebildet ist.

4. Querverbinder nach einem der vorhergehenden Ansprüche, **dadurch gekennzeichnet, dass** der Klemmdeckel (20) verschwenkbar am stabförmigen Abschnitt (12) gelagert ist.

5. Klemmdeckel nach einem der vorhergehenden Ansprüche, **dadurch gekennzeichnet, dass** der Bügel (22) aus der Ebene des Klemmdeckels (20) im Wesentlichen um 90° abgewinkelt ist und den stabförmigen Abschnitt (12) orthogonal zu dessen Längsachse umgreift.

6. Querverbinder nach einem der vorhergehenden Ansprüche, **dadurch gekennzeichnet, dass** der Bügel eine ein Lager (32) bildende Schulter (34) aufweist, die sich an einem Gegenlager (36) des anderen Teils (18) des Klemmabschnitts (14) abstützt.

7. Querverbinder nach Anspruch 6, **dadurch gekennzeichnet, dass** das Lager (32) mit Gegenlager (36) einerseits und eine Klemmschraube (30) andererseits auf gegenüberliegenden Seiten einer Aufnahmeöffnung (26) für den Fixierstab angeordnet sind.

8. Querverbinder nach einem der vorhergehenden Ansprüche, **dadurch gekennzeichnet, dass** der stabförmige Abschnitt (12) eine Oberflächenstruktur aufweist, insbesondere mit in Längsrichtung verlaufenden Nuten (16) versehen ist.

9. Querverbinder nach Anspruch 2 und einem der vorhergehenden Ansprüche, **dadurch gekennzeichnet, dass** die Aufnahmeöffnung (26) für den Fixierstab an ihrer Innenoberfläche zumindest abschnittsweise mit einer Oberflächenstruktur, insbesondere mit in Längsrichtung zur Öffnung verlaufenden Nuten (38) versehen ist.

10. Querverbinder nach Anspruch 2 und einem der vorhergehenden Ansprüche, **dadurch gekennzeichnet, dass** der Durchmesser des stabförmigen Abschnitts (12) dem Durchmesser der Aufnahmeöffnung (26) entspricht.

11. Querverbinder nach einem der vorhergehenden Ansprüche, **dadurch gekennzeichnet, dass** der Klemmabschnitt (14) mit einer verliersicher befestigten Schraube (30) versehen ist.

12. Querverbinder nach einem der vorhergehenden Ansprüche, **dadurch gekennzeichnet, dass** dieser aus Edelstahl oder Titan besteht.

## Claims

1. A transverse connector for osteosynthesis to capture a fixation rod interconnecting several vertebrae, the transverse connector (10) comprising a rod-shaped section (12) for mounting the connector and a clamping section (14) for connecting the fixation rod, and wherein the clamping section (14) is formed from two parts and has a clamping lid (20), **characterized in that** the clamping lid (20) comprising a shackle (22) surrounding said rod-shaped section (12).

2. Traverse connector according to claim 1, **characterized in that** the clamping section (14) comprising an opening (26) receiving the fixation rod, and said opening (26) having a slot (28), open at an edge thereof.

3. Traverse connector according to claim 1 or 2, **characterized in that** said clamping lid (20) defines a part of said opening (26) for the fixation rod.

4. Traverse connector according to one of the preceding claims, **characterized in that** said clamping lid (20) is pivotably disposed on said rod-shaped section (12).

5. Traverse connector according to one of the preceding claims, **characterized in that** said shackle (22) is bent through substantially 90° away from a plane of said clamping lid (20) and surrounds said rod-shaped section (12) orthogonally to a longitudinal axis thereof.

6. Traverse connector according to one of the preceding claims, **characterized in that** said shackle comprises a shoulder (34) forming a bearing (32) which is supported on a counter bearing (36) of the other part (18) of said clamping section (14).

7. Traverse connector according to claim 6, **characterized in that** said bearing (32) with counter bearing (36) on the one hand and a clamping screw (30) on the other hand are arranged on opposite sides of a receiving opening (26) for the fixation rod.

8. Traverse connector according to one of the preceding claims, **characterized in that** said rod-shaped section (12) has a surface structure, in particular with grooves (16) extending in a longitudinal direction.

9. Traverse connector according to claim 2, **characterized in that** at least sections of an inner surface of said receiving opening (26) for the fixation rod are provided with a surface structure, in particular with grooves (38) extending in a longitudinal direction of said opening.

10. Traverse connector according to claim 2, **characterized in that** the diameter of said rod-shaped section (12) corresponds to a diameter of said receiving opening (26).

11. Traverse connector according to one of the preceding claims, **characterized in that** said clamping section (14) comprises a securely mounted screw (30).

12. Traverse connector according to one of the preceding claims, **characterized in that** the connector is made from stainless steel or titanium.

## Revendications

1. Elément de connexion transversal pour l'ostéosynthèse, pour le raccordement d'une tige de fixation connectant plusieurs vertèbres les unes aux autre, l'élément de connexion transversal (10) présentant une section en forme de tige (12) pour sa fixation et une section de serrage (14) pour le raccordement de la tige de fixation, et la section de serrage (14) étant réalisée en deux parties avec un couvercle de serrage (20), **caractérisé en ce que** le couvercle de serrage (20) est muni d'un étrier (22) entourant la section en forme de tige (12).

2. Elément de connexion transversal selon la revendication 1, **caractérisé en ce que** la section de serrage (14) présente une ouverture de logement (26) pour la tige de fixation, et **en ce que** cette ouverture (26) est munie d'une fente (28) à bord ouvert.

3. Elément de connexion transversal selon la revendication 1 ou 2, **caractérisé en ce que** le couvercle de serrage (20) est réalisé avec une partie de l'ouverture de logement (26) pour la tige de fixation.

4. Elément de connexion transversal selon l'une des revendications précédentes, **caractérisé en ce que** le couvercle de serrage (20) est monté sur la section en forme de tige (12) de manière à pouvoir pivoter.

5. Couvercle de serrage selon l'une des revendications précédentes, **caractérisé en ce que** l'étrier (22) est incliné de sensiblement 90° par rapport au plan du couvercle de serrage (20) et enserre la section en forme de tige (12) orthogonalement par rapport à son axe longitudinal.

6. Elément de connexion transversal selon l'une des revendications précédentes, **caractérisé en ce que** l'étrier présente un épaulement (34) formant un palier (32), lequel épaulement prend appui sur un contre-palier (36) de l'autre partie (18) de la section de serrage (14).

7. Elément de connexion transversal selon la revendication 6, **caractérisé en ce que** le palier (32) est disposé, avec le contre-palier (36) d'une part et une vis de fixation (30) d'autre part, sur les faces opposées d'une ouverture de logement (26) pour la tige de fixation.

8. Elément de connexion transversal selon l'une des revendications précédentes, **caractérisé en ce que** la section en forme de tige (12) présente une structure superficielle, notamment est munie de rainures (16) orientées dans le sens longitudinal.

9. Elément de connexion transversal selon la revendication 2 et l'une des revendications précédentes, **caractérisé en ce que** l'ouverture de logement (26) pour la tige de fixation est munie sur sa surface intérieure au moins par sections d'une structure de surface, notamment de rainures (38) orientées dans le sens longitudinal de l'ouverture.

10. Elément de connexion transversal selon la revendication 2 et l'une des revendications précédentes, **caractérisé en ce que** le diamètre de la section en forme de tige (12) correspond au diamètre de l'ouverture de logement (26).

11. Elément de connexion transversal selon l'une des revendications précédentes, **caractérisé en ce que** la section de serrage (14) est munie d'une vis (30) imperdable.

12. Elément de connexion selon l'une des revendications précédentes, **caractérisé en ce qu'**il est constitué d'acier spécial ou de titane.
